# EUROPÄISCHE PATENTSCHRIFT

(11) **EP 2 086 624 B1**
(45) Veröffentlichungstag und Bekanntmachung des Hinweises auf die Patenterteilung: **21.12.2016**
(21) Anmeldenummer: 07819219.2
(22) Anmeldetag: 23.10.2007
(51) Int. Cl.: A61M 39/10

(54) **VERBINDUNGSSTÜCK FÜR EIN ENTERALES ÜBERLEITSYSTEM**
CONNECTION PIECE FOR AN ENTERAL TRANSFER SYSTEM
PIÈCE DE RACCORDEMENT POUR UN SYSTÈME DE TRANSFERT ENTÉRAL

(30) Priorität: 25.10.2006 DE 102006050212
(43) Veröffentlichungstag der Anmeldung: 12.08.2009
(73) Patentinhaber: Fresenius Kabi Deutschland GmbH, 61352 Bad Homburg v.d.H. (DE)
(72) Erfinder: BECKER, Michael, 75438 Knittlingen (DE)
(74) Vertreter: Kusche, Robert
(86) Internationale Anmeldenummer: PCT/EP2007/009156
(87) Internationale Veröffentlichungsnummer: WO 2008/049568

(56) Entgegenhaltungen:
- EP-A1- 0 158 030
- EP-A1- 0 633 039
- WO-A-02/02048
- WO-A-81/01654
- WO-A-91/19462
- WO-A2-01/17583
- WO-A2-95/13105
- DE-A1- 19 938 975
- US-A- 4 511 163
- US-A- 4 729 401
- US-A- 5 047 021
- US-A- 5 275 447
- US-A- 5 330 448
- US-A- 5 405 339
- US-A- 6 068 617
- US-A1- 2005 033 269
- US-B1- 6 971 390

## Beschreibung

Die Erfindung bezieht sich auf ein Verbindungsstück für ein enterales Überleitsystem, das ein distales Anschlussstück zum Anschluss eines Verbindungsstücks eines Sondenschlauchs einer enteralen Ernährungssonde und ein proximales Anschlussstück zum Anschluss eines Verbindungsschlauchs eines Überleitsystems aufweist. Darüber hinaus betrifft die Erfindung ein Überleitsystem für die enterale Ernährung mit einem derartigen Verbindungsstück. Des Weiteren betrifft die Erfindung eine Anordnung bestehend aus einem derartigen Überleitsystem und einer enteralen Ernährungssonde, die einen Sondenschlauch mit einem Verbindungsstück zum Anschluss des Überleitsystems aufweist.

Bei der enteralen Ernährung wird eine flüssige Nährlösung mit einer enteralen Ernährungssonde dem Patienten appliziert. Die enterale Nährlösung wird in einem Behältnis bereitgestellt, das über ein sogenanntes Überleitsystem mit der enteralen Ernährungssonde verbunden wird. Das enterale Überleitsystem weist einen Sondenschlauch mit einem distalen und proximalen Verbindungsstück auf. Das distale Verbindungsstück wird mit der Ernährungssonde und das proximale Verbindungsstück mit dem Nährlösungsbehältnis verbunden, so dass die Nährlösung aus dem Behältnis über den Verbindungsschlauch des Überleitsystems in die Ernährungssonde fließt.

Das Nährlösungsbehältnis wird in der Regel mehrmals täglich ausgetauscht, während beim Überleitsystem ein Wechsel alle 24 Stunden empfohlen wird. Die Ernährungssonde kann bis zu mehreren Monaten verwendet werden.

Zur Verbindung des Verbindungsschlauchs des Überleitsystems mit dem Sondenschlauch der Ernährungssonde finden im Stand der Technik die bekannten Luer-Lock-Konnektoren Verwendung, so dass Überleitsystem und Ernährungssonde miteinander verbunden werden können, ohne dass die Gefahr besteht, dass sich der Sondenschlauch der Ernährungssonde von dem Verbindungsschlauch des Überleitsystems während der Applikation der Nährlösung unbeabsichtigt löst. Nachteilig ist jedoch, dass die bekannten Luer-Lock-Verbindungen in der enteralen Applikationstechnik insofern ein Risiko für den Patienten darstellen können, als die Luer-Lock-Verbindungen in der enteralen Applikationstechnik mit Luer-Lock-Verbindungen verwechselt werden können, die insbesondere in der intravenösen Applikation und in der Beatmungstechnik eingesetzt werden. Wenn beispielsweise eine enterale Ernährung über eine intravenös gelegte Luer-Lock-Verbindung versehentlich appliziert werden sollte, besteht ein erhebliches Risiko für den Patienten.

Ein Sicherheitsgewinn wird im Stand der Technik dadurch erreicht, dass das enterale Überleitsystem ein distales weibliches Luer-Lock-Verbindungsstück aufweist, während die enterale Ernährungssonde ein männliches Luer-Lock-Verbindungsstück aufweist. Da intravenöse Sonden aber über ein weibliches Luer-Lock-Verbinungsstück verfügen, ist ausgeschlossen, dass ein enterales Überleitsystem direkt mit der intravenösen Sonde verbunden werden kann. Aufgrund der Vielzahl von verfügbaren Luer-Lock-Adaptern, die von den verschiedenen Herstellern angeboten werden, sind aber Verwechselungen nicht gänzlich auszuschließen.

Neben den Überleitsystemen und Ernährungssonden mit Luer-Lock-Konnektoren sind Ernährungssonden bekannt, die über ein trichterförmiges Verbindungsstück verfügen. Dabei bieten die verschiedenen Hersteller Ernährungssonden mit Anschlusstrichtern unterschiedlichen Durchmessers an. Um eine Verbindung mit den Anschlusstrichtern der bekannten Ernährungssonden herstellen zu können, sind im Stand der Technik stufenförmig ausgebildete Adapter bekannt, die im allgemeinen über eine Luer-Lock-Verbindung mit dem Verbindungsschlauch des Überleitsystems lösbar verbunden werden.

Die bekannten Stufenadapter haben den Vorteil, dass eine Verbindung mit Anschlusstrichtern unterschiedlichen Durchmessers möglich ist. Der Stufenadapter wird in den Anschlusstrichter der Ernährungssonde gesteckt, wobei in Abhängigkeit von dem Durchmesser des Anschlusstrichters derjenige Abschnitt des Stufenadapters, dessen Durchmesser dem Durchmesser des Anschlusstrichters entspricht, mit dem Anschlusstrichter in Kontakt kommt. Dabei werden die beiden Teile durch die Reibungskraft der Mantelflächen von Stufenadapter und Anschlusstrichter fixiert.

Während der Stufenadapter im allgemeinen aus einem härteren Material hergestellt ist, besteht der Anschlusstrichter in der Regel aus weicherem Material, um den Kraftschluss zu verbessern. Nachteilig ist, dass die Haltekraft zwischen Stufenadapter und Anschlusstrichter nicht ausreichend sein kann. Insbesondere bei der Verwendung von fetthaltigen Flüssignahrungen besteht das Risiko, dass sich die Verbindung unbeabsichtigt löst. Auch Alterungseffekte, die auf die Materialeigenschaften des Anschlusstrichters und seine Geometrie Einfluss haben, können zu einer reduzierten Haltekraft führen.

Schraubverbindungen, die auch im Bereich der enteralen Ernährung Verwendung finden, sind aus der WO 2005/055919 A1 bekannt. Nachteilig ist, dass die bekannten Schraubverbindungen nicht universell einsetzbar sind, sondern an die jeweilige Ernährungssonde bzw. das Überleitsystem, die über entsprechende Verbindungen verfügen, gebunden sind.

Ein stufenförmig ausgebildetes Anschlussstück mit mehreren Abschnitten unterschiedlichen Durchmessers, auf das ein elastischer Schlauch aufgeschoben wird, ist beispielsweise aus der US-A-4 693 707 bekannt. Mit dem bekannten Stufenadapter kann zwar eine dichte Verbindung mit Schläuchen unterschiedlichen Durchmessers hergestellt werden, nachteilig ist jedoch, dass die Gefahr besteht, dass sich der Schlauch bei Zugbeanspruchung von dem Verbindungsstück löst.

Ein Stufenadapter zur Verwendung im Bereich der enteralen Ernährung ist aus der WO 02/051494 A1 bekannt. Auch hier besteht das Risiko des unbeabsichtigten Lösen des Sondenschlauchs der Ernährungssonde.

Die US-A-4 692 150 beschreibt eine Anordnung, die einen Sondenschlauch mit einem Anschlusstrichter und einen Verbindungsschlauch mit einem Stufenadapter umfasst. Zur Sicherung des Stufenadapters in dem Anschlusstrichter schlägt die Druckschrift die Verwendung eines Sicherungsbandes vor, das nach dem Zusammenstecken der beiden Teile über die Verbindungsstelle geschoben wird, so dass die beiden Teile zusammengehalten werden. Gleichsam soll das Sicherungsband als Schutz vor Bakterien dienen. Nachteilig ist jedoch, dass die Verwendung eines zusätzlichen Sicherungsbandes, das nach dem Zusammenstecken der Teile über die Verbindungsstelle geschoben werden muss, die Handhabung US 20050033269, offenbart die Präambel von Anspruch 1.

Der Erfindung liegt die Aufgabe zugrunde, ein universell einsetzbares und einfach zu handhabendes Verbindungsstück für ein enterales Überleitsystem bereit zu stellen, das eine sichere, aber lösbare Verbindung von Überleitsystem und Ernährungssonde ermöglicht. Eine weitere Aufgabe der Erfindung liegt darin, ein universell einsetzbares und einfach zu handhabendes Überleitsystem für die enterale Ernährung zu schaffen, das mit einer Ernährungssonde verbunden werden kann, ohne dass die Gefahr des unbeabsichtigten Lösens der Verbindung besteht. Darüber hinaus ist eine Aufgabe der Erfindung, eine universell einsetzbare und einfach zu handhabende Anordnung bestehend aus einem Überleitsystem und einer Ernährungssonde bereit zu stellen, wobei sich Ernährungssonde und Überleitsystem sicher miteinander verbinden lassen.

Die Lösung dieser Aufgaben erfolgt erfindungsgemäß mit den in den Patentansprüchen 1, 12 und 14 angegebenen Merkmalen. Vorteilhafte Ausführungsformen sind Gegenstand der Unteransprüche.

Das erfindungsgemäße Verbindungsstück für ein enterales Überleitsystem verfügt über ein distales Anschlussstück zum Anschluss eines Verbindungsstücks eines Sondenschlauchs einer enteralen Ernährungssonde und ein proximales Anschlussstück zum Anschluss des Verbindungsschlauchs eines Überleitsystems.

Das distale Anschlussstück des Verbindungsstücks weist entweder einen konischen Abschnitt oder einen stufenförmigen Abschnitt auf. Dabei kann der stufenförmige Abschnitt des distalen Anschlussstücks selbst wieder konische und/oder zylindrische Absätze unterschiedlichen Durchmessers aufweisen. Entscheidend ist, dass der konische oder stufenförmige Abschnitt des distalen Anschlussstücks sich verjüngt. Daher kann das distale Anschlussstück mit den trichterförmigen Verbindungsstücken unterschiedlichen Durchmessers der Sondenschläuche von den bekannten Ernährungssonden lösbar verbunden werden. Insofern ist das erfindungsgemäße Verbindungsstück universell einsetzbar.

Das distale Anschlussstück des erfindungsgemäßen Verbindungsstücks weist ein Arretierungselement auf, das derart ausgebildet ist, dass eine formschlüssige lösbare Verbindung mit einem Verbindungsstück eines Sondenschlauchs einer enteralen Ernährungssonde herstellbar ist, das ein mit dem Arretierungselement des distalen Anschlussstücks komplementäres Arretierungselement aufweist. Das Arretierungselement kann unterschiedlich ausgebildet sein. Als Arretierungselemente kommen grundsätzlich alle formschlüssigen lösbaren Verbindungen in Frage, beispielsweise Steckverbindungen und Schraubverbindungen, insbesondere Überwurfkappen, Schraubverschlüsse, BajonettVerschlüsse oder dergleichen.

Mit der besonderen Ausbildung des distalen Anschlussstücks ist also einerseits die Herstellung einer formschlüssigen lösbaren Verbindung mit einem Verbindungsstück eines Sondenschlauchs einer enteralen Ernährungssonde möglich, das ein mit dem Arretierungselement des distalen Anschlussstücks komplementäres Arretierungselement aufweist, andererseits kann das erfmdungsgemäße Verbindungsstück in bekannter Weise mit den trichterförmigen Verbindungsstücken unterschiedlichen Durchmessers der bekannten Ernährungssonden verbunden werden.

Von Vorteil ist, dass das erfindungsgemäße Verbindungsstück Verwechselungen mit bei nicht enteralen Zugängen eingesetzten Luer-Lock-Verbindungen wegen der Inkompatibilität der Verbindungsstücke ausschließt.

Das erfindungsgemäße Verbindungsstück schafft für den Fall, dass das Verbindungsstück zum Anschluss einer Ernährungssonde eingesetzt wird, die über ein komplementäres Arretierungselement verfügt, eine leicht lösbare sowohl kraft- als auch formschlüssige Verbindung, die gegen unbeabsichtigtes Lösen gesichert ist.

Die universelle Einsetzbarkeit des erfindungsgemäßen Verbindungsstücks führt zu einer Kostenreduktion bei der Bereitstellung unterschiedlicher Ernährungssonden und bei Überleitsystemen. Weiterhin ist die Umstellung von den bekannten Ernährungssonden mit Anschlusstrichter auf Ernährungssonden, die über ein komplementäres Arretierungselement verfügen, mit geringeren Problemen verbunden, weil zumindest für eine gewisse Übergangszeit der Anschluss konventioneller Ernährungssonden noch möglich ist.

Das erfindungsgemäße Verbindungsstück vereint die Vorteile der bekannten Luer-Lock-Konnektoren mit den Vorteilen der nicht formschlüssigen, sondern nur kraftschlüssigen Verbindungen von trichterförmigen Anschlussstücken und Stufenadaptern oder auch konischen Adaptern, ohne jedoch deren Nachteile in Kauf zu nehmen.

Bei einer bevorzugten Ausführungsform des erfindungsgemäßen Verbindungsstücks ist der vordere Abschnitt des distalen Anschlussstücks mit dem kleinsten Durchmesser als Arretierungselement ausgebildet. Die Abdichtung der Verbindungsstücke von Ernährungssonde und Überleitsystem erfolgt bei dieser Ausführungsform insbesondere an der Stirnfläche des distalen Anschlussstücks, das dichtend an der Stirnfläche des Verbindungsstücks der Ernährungssonde liegt. Es ist grundsätzlich aber auch möglich, das Arretierungselement im hinteren oder mittleren Bereich des entweder konischen oder in mehreren Abschnitten stufenförmigen distalen Anschlussstücks vorzusehen.

Bei einer alternativen besonders bevorzugten Ausführungsform ist der vordere Abschnitt des distalen Anschlussstücks als Verbindungskonus und der sich an den Verbindungskonus anschließende Abschnitt des distalen Anschlussstücks als Arretierungselement ausgebildet. Die Abdichtung erfolgt hier dadurch, dass der Verbindungskonus des Anschlussstücks in eine Bohrung des Verbindungsstücks der Ernährungssonde greift, die eine entsprechende Konizität aufweist.

In beiden Fällen wird eine dichte, aber leicht lösbare Verbindung von Sondenschlauch und Verbindungsschlauch der Ernährungssonde bzw. des Überleitsystems geschaffen.

Eine besondere Ausführungsform sieht vor, dass das Arretierungselement des distalen Anschlussstücks als Schraubverbindungselement, vorzugsweise als ein zylindrisches oder konisches Verbindungselement mit einem Außengewinde ausgebildet ist. Es ist aber auch möglich, dass das Schraubverbindungselement ein Innengewinde aufweist. Beispielsweise kann ein zylindrischer Abschnitt mit einem spiralförmig umlaufenden Steg oder einer spiralförmigen Nut vorgesehen sein. Auch kann der Abschnitt konisch sein, wobei die Höhe des Steges oder die Tiefe der Nut über die Länge des Abschnitts derart bemessen ist, dass der Abstand zwischen dem Nutgrund bzw. der Außenseite des Steges und der Achse des zylindrischen oder konischen Abschnitts über die Länge gleich bleibt. Das Verbindungsstück des Sondenschlauchs ist dann jeweils komplementär ausgebildet.

Eine weitere Ausführungsform sieht vor, dass das Arretierungselement des distalen Anschlussstücks als Bajonett-Verbindungselement ausgebildet ist. Vorzugsweise ist das Bajonett-Verbindungselement ein zylindrisches Verbindungselement, dessen Mantelfläche eine Nut, vorzugsweise mit einem axial verlaufenden Abschnitt aufweist, an den sich ein über einen Teil des Umfangs der Mantelfläche verlaufender Abschnitt anschließt. Es ist aber auch möglich, dass das zylindrische Verbindungselement das hierzu komplementäre Bajonett-Verbindungselement ist, das einen in eine Nut greifenden Ansatz aufweist.

Grundsätzlich können alle dem Fachmann als Bajonett-Verbindungen bekannten Verbindungstechniken vorgesehen werden. Das Bajonett-Verbindungselement kann grundsätzlich im hinteren, mittleren oder vorderen Bereich des distalen Anschlussstücks vorgesehen sein. Das Verbindungsstück des Sondenschlauchs ist dann wieder jeweils komplementär ausgebildet.

Bei einer alternativen besonders bevorzugten Ausführungsform ist das Arretierungselement des distalen Anschlussstücks nicht als ein Schraubverbindungselement an dem stufenförmigen oder konischen Abschnitt des distalen Anschlussstücks, sondern als eine Schraubkappe mit einem Innengewinde ausgebildet, die den konischen Abschnitt des distalen Anschlussstücks zumindest über einen Teil seiner Länge umschließt, aber auch einen stufenförmigen Abschnitt zumindest über einen Teil seiner Länge umschließen kann, wenn das distale Anschlussstück anstelle eines konischen einen stufenförmigen Abschnitt aufweist. Die Schraubkappe kann einstückiger Bestandteil des distalen Anschlusstücks oder um die Achse des distalen Anschlussstücks drehbar sein.

Anstelle einer Schraubkappe kann aber auch eine Kappe vorgesehen sein, die passend auf das Verbindungsstück des Sondenschlauchs aufgesetzt wird. Diese kann beispielsweise klemmend oder einrastend an dem Verbindungsstück des Sondenschlauchs fixiert sein.

Die Kappe kann auch als Bajonettverschluss ausgebildet sein, wobei entweder der in die Nut greifende Absatz oder die Nut des Bajonettverschlusses an dem distalen Verbindungsstück vorgesehen sein kann.

Es versteht sich von selbst, dass das Verbindungsstück des Sondenschlauchs bei den unterschiedlichen Ausführungsformen des distalen Verbindungsstücks wieder komplementär ausgebildet ist. Beispielsweise kann das Verbindungsstück des Sondenschlauchs als Anschlusstrichter ausgebildet sein, wobei an der Außenseite des Trichters ein Außengewinde für eine Schraubverbindung oder das jeweils komplementäre Gegenstück eines Bajonettverschlusses vorgesehen sein kann.

Das proximale Anschlussstück des erfindungsgemäßen Verbindungsstücks kann als ein Anschlussstück zur Herstellung einer lösbaren Verbindung mit einem komplementären Anschlussstück des Überleitsystems, insbesondere als ein Luer-Lock-Anschlussstück ausgebildet sein. Dadurch ist es möglich, das erfindungsgemäße Verbindungsstück so zu handhaben und zu verwenden, wie die bekannten Stufenadapter, die an die Luer-Lock-Anschlussstücke der bekannten Überleitsysteme angeschlossen werden. Vorzugsweise wird das erfindungsgemäße Verbindungsstück aber direkt mit dem Sondenschlauch der Ernährungssonde verbunden, beispielsweise verklebt und/oder verschweißt.

Das erfindungsgemäße Überleitsystem umfasst das erfindungsgemäße Verbindungsstück und ein enterales Überleitsystem, wobei das erfindungsgemäße Verbindungsstück als Adapter für das Luer-Lock-Anschlussstück des enteralen Überleitsystems ausgebildet oder fest mit dem Verbindungsschlauch des enteralen Überleitsystems verbunden sein kann.

Die erfindungsgemäße Anordnung umfasst das erfindungsgemäße Überleitsystem und eine enterale Ernähmngssonde, die an das Verbindungsstück des erfindungsgemäßen Überleitsystems angeschlossen werden kann.

Im Folgenden werden verschiedene Ausfiihrungsbeispiele der Erfindung unter Bezugnahme auf die Zeichnungen näher erläutert.

Es zeigen:
- Fig. 1: eine Anordnung bestehend aus einem Überleitsystem und einer enteralen Ernährungssonde,
- Fig. 2a und 2b: das Verbindungsstück des Überleitsystems und der Ernährungssonde von Fig. 1 in vergrößerter Darstellung vor und nach dem Herstellen einer Verbindung zwischen dem Überleitsystem und der Ernährungssonde,
- Fig. 3a und 3b: das Verbindungsstück des Überleitsystems von Fig. 1 und ein trichterförmiges Verbindungsstück einer bekannten Ernährungssonde in vergrößerter Darstellung vor und nach dem Herstellen einer Verbindung zwischen Überleitsystem und Ernährungssonde,
- Fig. 4a und 4b: das Verbindungsstück des Überleitsystems von Fig. 1 und ein trichterförmiges Verbindungsstück einer anderen bekannten Ernährungssonde, wobei das Verbindungsstück einen kleineren Durchmesser als das Verbindungsstück der Ernährungssonde von Fig. 3a und 3b aufweist,
- Fig. 5a und Fig. 5b: eine nicht erfindungsgemässen Ausführungsform der Verbindungsstücke des Überleitsystems und der Ernährungssonde,
- Fig. 6: eine weitere alternative Ausführungsform des Verbindungsstücks des Überleitsystems,
- Fig. 7: eine nicht erfindungsgemässen Ausführungsform des Verbindungsstücks des Überleitsystems,
- Fig. 8a und 8b: das Verbindungsstück der Emährungssonde von Fig. 1 und ein Adapter für das Verbindungsstück in vergrößerter Darstellung vor und nach der Herstellung einer Verbindung von Verbindungsstück und Adapter,
- Fig. 9: ein weiteres Ausführungsbeispiel des erfindungsgemäßen Verbindungsstücks zusammen mit dem Verbindungsstück des Sondenschlauchs und
- Fig. 10: ein Ausführungsbeispiel eines nicht erfindungsgemäßen Verbindungsstücks zusammen mit dem Verbindungsstück des Sondenschlauchs.

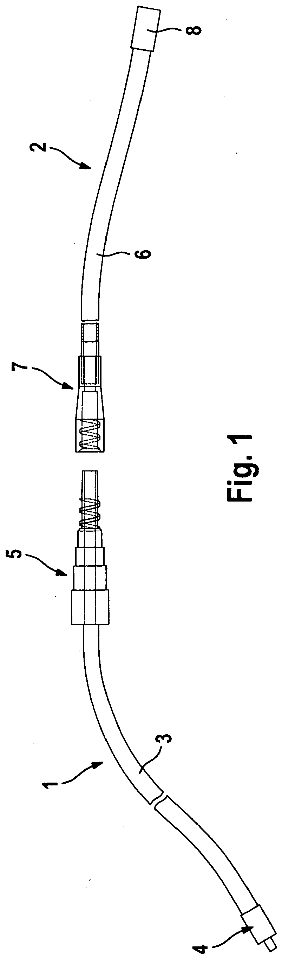

Fig. 1 zeigt in vereinfachter Darstellung eine Anordnung bestehend aus einem Überleitsystem 1 für eine enterale Ernährung und einer enteralen Ernährungssonde 2, die miteinander verbunden werden, um eine enterale Nährlösung aus einem nicht dargestellten Nährlösungsbehältnis dem Patienten zu applizieren.

Da enterale Überleitsysteme und Ernährungssonden als solche dem Fachmann bekannt sind, beschränkt sich die Beschreibung im Wesentlichen auf die für die Erfindung relevanten Teile.

Das Überleitsystem 1 weist einen Verbindungsschlauch 3 mit einem proximalen Verbindungsstück 4 am proximalen Schlauchende und einem distalen Verbindungsstück 5 am distalen Schlauchende auf. Das proximale Verbindungsstück 4 dient dem Anschluss des Überleitsystems 1 an ein nicht dargestelltes Nährlösungsbehältnis, während das distale Verbindungsstück 5 dem Anschluss der Ernährungssonde 2 dient.

Die Ernährungssonde 2 weist einen Sondenschlauch 6 mit einem proximalen Verbindungsstück 7 am proximalen Schlauchende und einem in den Patienten einzuführenden Endstück 8 am distalen Schlauchende auf. Das proximale Verbindungsstück 7 der Ernähmngssonde 2 wird mit dem distalen Verbindungsstück 5 des Überleitsystems 1 verbunden.

Im Folgenden werden unterschiedliche Ausführungsbeispiele der Verbindungsstücke von Überleitsystem 1 und Ernährungssonde 2 im Einzelnen beschrieben.

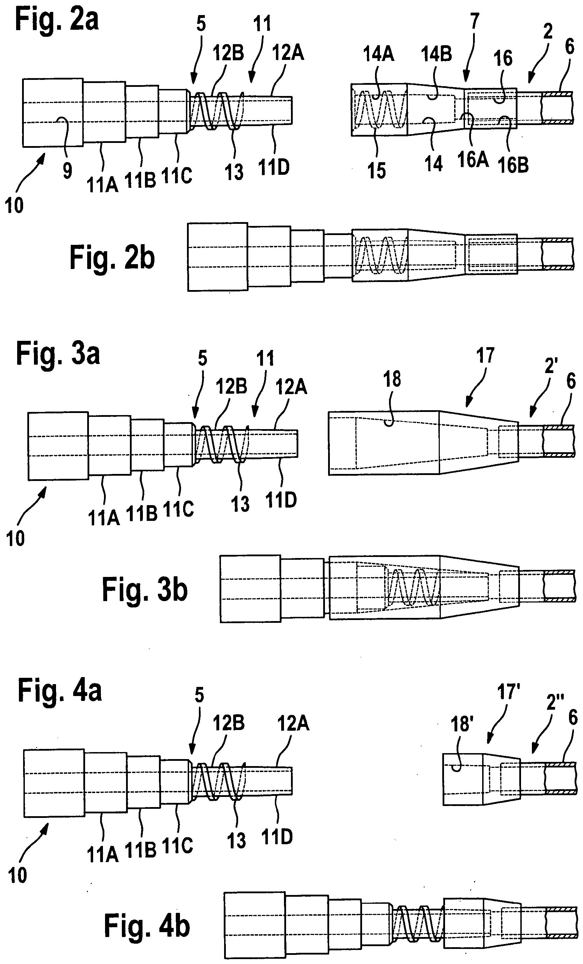

Die Figuren 2a und 2b zeigen ein erstes Ausführungsbeispiel des proximalen Verbindungsstücks 5 des Überleitsystems 1 und des distalen Verbindungsstücks 7 der Ernährungssonde 2 in vergrößerter Darstellung. Fig. 2a zeigt die Verbindungsstücke vor dem Verschrauben und Fig. 2b nach dem Verschrauben.

Das Verbindungsstück 5 ist ein rohrförmiges Kunststoffteil, das im Spritzgießverfahren hergestellt ist. Der in dem Verbindungsstück ausgebildete Kanal 9 ist in gestrichelten Linien dargestellt. Das Verbindungsstück 5 weist ein proximales Anschlussstück 10 auf, das in den Figuren 2a und 2b das linke Teilstück des Verbindungsstücks darstellt. Mit dem proximalen Anschlussstück 10 ist das distale Schlauchende des Verbindungsschlauchs 3 des Überleitsystems 1 fest verbunden ist. Hierzu kann das Schlauchende beispielsweise in eine Bohrung des proximalen Anschlussstücks 10 eingesetzt und mit dem Anschlussstück verklebt und/oder verschweißt sein.

Zum Anschluss der Ernährungssonde 2 weist das Verbindungsstück des Überleitsystems 1 ein distales Anschlussstück 11 auf, das in den Figuren 2a und 2b das rechte Teilstück des Verbindungsstücks darstellt. Das distale Anschlussstück 11 ist ein stufenförmiges Teilstück mit mehreren, bei den vorliegenden Ausführungsbeispiel insgesamt vier Abschnitten 11A, 11B, 11C, 11D unterschiedlichen Durchmessers, wobei der Durchmesser zum vorderen Ende hin abnimmt. Dabei hat der vordere Abschnitt 11D etwa die doppelte Länge wie die hinteren Abschnitte 11A, 11B, 11C.

Der vordere Abschnitt 11D umfasst ein vorderes konisches Teilstück 12A und ein hinteres zylindrisches Teilstück 12B, das mit einem Außengewinde 13 versehen ist. Das konische Teilstück bildet einen Verbindungskonus, der in eine Bohrung entsprechender Konizität des Verbindungsstücks 7 der Ernährungssonde 2 eingeschoben werden kann, während das zylindrische Teilstück 12B mit dem Außengewinde 13 ein Arretierungselement bildet, so dass eine formschüssige Verbindung mit einem komplementären Arretierungselement des Verbindungsstücks 7 der Ernährungssonde 2 hergestellt werden kann.

Das Verbindungsstück 7 der Ernährungssonde 2 ist ebenfalls ein rohrförmiges Kunststoffteil, das im Spritzgießverfahren hergestellt ist. Das Verbindungsstück 7 weist eine in gestrichelten Linien dargestellte Bohrung 14 mit einem vorderen zylindrischen Abschnitt 14A und einem hinteren leicht konischen Abschnitt 14B auf. Der zylindrische Abschnitt 14A der Bohrung 14 des Verbindungsstücks 7 weist ein in gestrichelten Linien dargestelltes Innengewinde 15 auf, das dem Außengewinde 13 des Verbindungsstücks 5 entspricht, und der zylindrische Abschnitt 14A hat einen Durchmesser, der dem Außendurchmesser des zylindrischen Teilstücks 12B des Verbindungsstücks 5 entspricht. Der konische Abschnitt 14B der Bohrung 14 hat eine Konizität, die der Konizität des konischen Teilstücks 12A des Verbindungsstücks 5 entspricht.

An die Bohrung 14 des Verbindungsstücks 7 schließt sich eine Bohrung 16 mit einem Teilstück 16A kleineren Durchmessers und einem Teilstück 16B größeren Durchmessers an. In dem Teilstück 16B sitzt der Sondenschlauch 6 der Ernährungssonde 2, die mit dem Verbindungsstück 7 vorzugsweise verklebt und/oder verschweißt ist.

Zur Herstellung einer Verbindung zwischen Überleitsystem 1 und Ernährungssonde 2 werden die jeweiligen Verbindungsstücke 5 und 7 zusammengesteckt und miteinander verschraubt. Dabei dichtet die Mantelfläche des konischen Teilstücks 12A gegenüber der Mantelfläche des konischen Abschnitts 14B ab, während die Schraubverbindung verhindert, dass sich die Verbindungsstücke unbeabsichtigt lösen können. Vorteilhaft ist auch, dass die kraft- und formschlüssige Verbindung alterungsbeständig ist, da sich Materialermüdungen oder -abnutzungen nur langfristig auswirken können.

Mit den Verbindungsstücken 5 und 7 kann sowohl eine kraftschlüssige als auch formschlüssige Verbindung geschaffen werden. Wenn der Durchmesser des vorderen Abschnitts 11D des Verbindungsstücks 5 so groß gewählt ist, dass das Verbindungsstück mit den weiblichen Luer-Konnektoren der nicht zur enteralen Ernährung bestimmten Katheter nicht kompatibel ist, können Verwechselungen ausgeschlossen werden.

Von Vorteil ist, dass das Verbindungsstück 5 des Überleitsystems 1 nicht nur mit dem passenden Verbindungsstück 7 der Ernährungssonde 2 verbunden werden kann, das unter Bezugnahme auf die Figuren 2a und 2b beschrieben ist, sondern gleichsam auch mit trichterförmigen Verbindungsstücken unterschiedlichen Durchmessers der bekannten Ernährungssonden.

Die Fig. 3a und 3b zeigen die Verbindung des Verbindungsstücks 5 des Überleitsystems mit einem trichterförmigen Verbindungsstück 17 einer bekannten Ernährungssonde 2'. Das trichterförmige Verbindungsstück 17 weist eine trichterförmige Bohrung 18 auf, die in gestrichelten Linien dargestellt ist. Die Verbindungsstücke 5 und 17 werden soweit zusammengesteckt bis das Verbindungsstück 5 fest in der trichterförmigen Bohrung 18 des Verbindungsstücks 17 sitzt. Dabei dichtet die Mantelfläche mindestens einer der Abschnitte 11A, 11B, 11C, 11D gegenüber der Mantelfläche der trichterförmigen Bohrung 18 ab. Gegenüber der Schraubverbindung hat diese Verbindung aber den Nachteil, dass ein unbeabsichtigtes Lösen der Verbindungsstücke möglich ist, wenn die Verbindung auf Zug beansprucht wird.

Die Figuren 4a und 4b machen deutlich, dass das Verbindungsstück 5 des Überleitsystems 1 auch mit einem trichterförmigen Verbindungsstück 17' einer bekannten Ernährungssonde 2" möglich ist, das sich in der Dimensionierung von dem Verbindungsstück 17 der Ernährungssonde unterscheidet, die in den Figuren 3a und 3b beschrieben ist. Die Sonde 2" hat ein kleineres Verbindungsstück 17' als die Sonde 2'. Über ein derartiges Verbindungsstück 17' verfügen beispielsweise die bekannten Pädiatrie-Sonden. Bei der in den Figuren 4a und 4b gezeigten Verbindung sitzt das konische Teilstück 12A des Verbindungsstücks 5 in der trichterförmigen Bohrung 18' des Verbindungsstücks 17'.

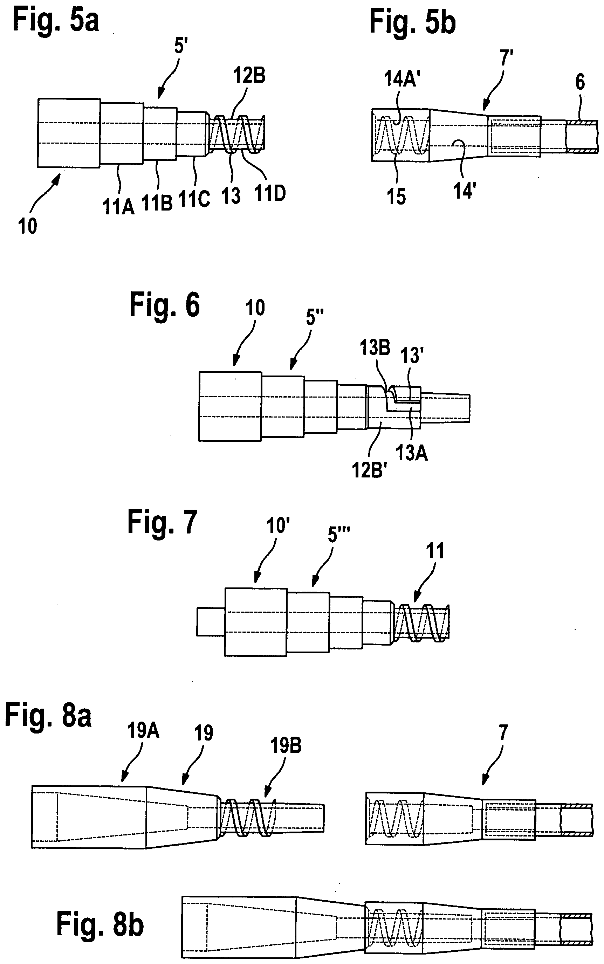

Die Figuren 5a und 5b zeigen ein weiteres Ausführungsbeispiel des Verbindungsstücks 5' des Überleitsystems 1, das sich von den unter Bezugnahme auf die Figuren 2a und 2b beschriebenen Ausführungsbeispiel nur dadurch unterscheidet, das der vordere Abschnitt 11D des Verbindungsstücks nicht das konische Teilstück 12A, sondern nur das zylindrische Teilstück 12B mit dem Außengewinde 13 aufweist. Ansonsten sind die Verbindungsstücke 5, 5' identisch. Daher sind die einander entsprechenden Teile auch mit denselben Bezugszeichen bezeichnet.

Das Verbindungsstück 5' des Überleitsystems 1 wird mit dem Verbindungsstück 7' der Ernährungssonde verbunden, das in Fig. 5b gezeigt ist. Das Verbindungsstück 7' unterscheidet sich von dem in den Figuren 2a und 2b gezeigten Verbindungsstück 7 dadurch, dass die Bohrung 14' nur das Teilstück 14A' mit dem Innengewinde 15, nicht aber das konische Teilstück aufweist. Grundsätzlich ist es aber auch möglich, das Verbindungsstück 5' mit dem Verbindungsstück 7 von Fig. 2a und 2b zu verschrauben.

Bei dem Ausführungsbeispiel von Fig. 5a und Fig. 5b erfolgt die Abdichtung durch Verpressen der Stirnflächen des Verbindungsstücks 7' des Sondenschlauchs 6 und des Verbindungsstücks 5' des Überleitsystems 1 beim Zusammenschrauben der Verbindungsstücke. Es ist aber grundsätzlich auch möglich, eine Abdichtung über das Gewinde vorzunehmen.

Fig. 6 zeigt ein weiteres Ausführungsbeispiel des Verbindungsstücks 5" des Überleitsystems 1, das sich von dem Ausführungsbeispiel der Figuren 2a und 2b dadurch unterscheidet, dass das zylindrische Teilstück 12B' als Bajonett-Verbindungselement ausgebildet ist, das anstelle des Außengewindes 13 in der Mantelfläche des zylindrischen Teilstücks eine Nut 13' aufweist, die einen axial verlaufenden Abschnitt 13A hat, an den sich ein über einen Teil des Umfangs der Mantelfläche verlaufender Abschnitt 13B anschließt. Das Verbindungsstück S" kann mit einem Verbindungsstück einer Ernährungssonde kraft- und formschlüssig verbunden werden, das anstelle eines Innengewindes über einen Ansatz an der Innenseite der Bohrung verfügt, welcher in die Nut 13' eingreift. Hierzu werden die Verbindungsstücke von Überleitsystem und Ernährungssonde zunächst in axialer Richtung zusammengeschoben und dann gegeneinander verdreht.

Fig. 7 zeigt ein weiteres Ausführungsbeispiel des Verbindungsstücks 5''' des Überleitsystems 1, das sich von dem Ausführungsbeispiel der Fig. 5a nur dadurch unterscheidet, dass das proximale Anschlussstück 10 für das distale Schlauchende des Verbindungsschlauchs 3 des Überleitsystems 1 nicht als Anschlussstück ausgebildet ist, mit dem der Verbindungsschlauch fest verbunden, beispielsweise verschweißt oder verklebt ist, sondern als Luer-Lock-Anschlussstück 10' ausgebildet ist, an das ein komplemäntäres Luer-Lock-Anschlussstück am distalen Schlauchende des Verbindungsschlauchs des Überleitsystems angeschlossen werden kann. Derartige komplementäre Luer-Lock-Anschlussstücke, die als männliche und weibliche Luer-Lock-Anschlussstücke bezeichnet werden, sind als solche dem Fachmann allgemein bekannt.

Für den Fall, dass eine Ernährungssonde Verwendung finden sollte, die über das in den Figuren 2a und 2b gezeigte Verbindungsstück 7 verfügt, und das erfindungsgemäße Verbindungsstück für das Überleitsystem nicht zur Verfügung stehen sollte, kann ein Überleitsystem mit einem herkömmlichen Verbindungsstück dann an das Verbindungsstück 7 der Ernährungssonde angeschlossen werden, wenn der in den Figuren 8a und 8b gezeigte Adapter 19 verwendet wird. Der Adapter 19 weist ein trichterförmiges Adapterstück 19A auf, das dem Adapter der bekannten Ernährungssonden entspricht, und weist ein zylindrisches Adapterstück 19B auf, das dem vorderen Teilstück 11D mit dem zylindrischen Abschnitt 12B mit Außengewinde 13 und dem konischen Abschnitt 12A entspricht (Fig. 2a und 2b). Wenn der Adapter 19 mit dem Verbindungsstück 7 verschraubt wird, kann die Ernährungssonde an die bekannten Überleitsysteme mit den herkömmlichen Verbindungsstücken angeschlossen werden.

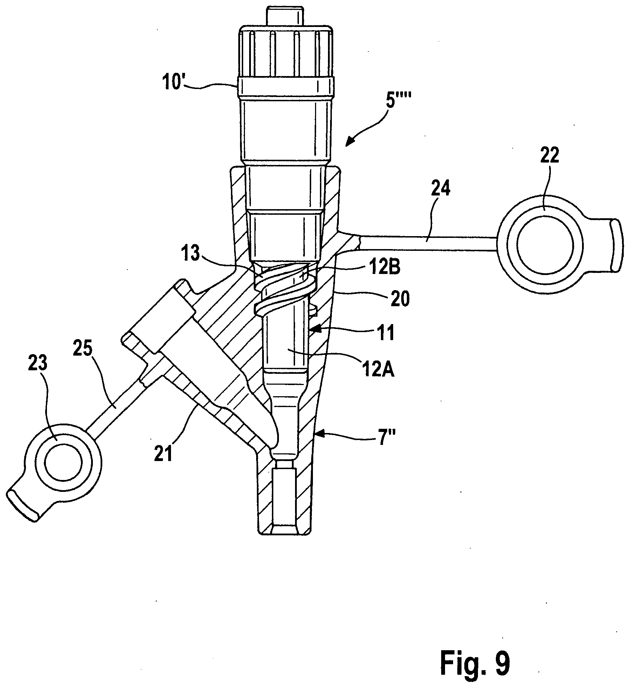

Fig. 9 zeigt ein weiteres Ausführungsbeispiel des erfindungsgemäßen Verbindungsstücks des Überleitsystems zusammen mit dem Verbindungsstück des Sondenschlauchs in teilweise geschnittener Darstellung. Das Verbindungsstück 5""des Überleitsystems weist für den Anschluss des distalen Schlauchendes des Verbindungsschlauchs des Überleitsystems wie das Ausführungsbeispiel von Fig. 7 ein Luer-Lock-Anschlussstück 10' auf. Von dem Ausführungsbeispiel von Fig. 7 unterscheidet sich die Ausführungsform von Fig. 9 dadurch, dass der als Arretierungselement 11 ausgebildete Abschnitt mit dem kleinsten Durchmesser nicht nur ein zylindrisches Verbindungselement 12B mit einem Außengewinde 13, sondern auch einen distalen Verbindungskonus 12A aufweist, der sich an das Verbindungselement anschließt.

Das Verbindungsstück 7" des Sondenschlauchs weist einen ersten Anschlusstrichter 20 auf, von dem ein zweiter Anschlusstrichter 21 abzweigt. Der erste Anschlusstrichter 20 dient dem Anschluss des Verbindungsstücks 5'''' des Überleitsystems, während der zweite Anschlusstrichter 21 dem Anschluss eines weiteren Anschlussstücks dient, das aber in Fig. 9 nicht dargestellt ist. Zum Verschließen der Anschlusstrichter sind Verschlusskappen 22, 23 vorgesehen, die über Laschen 24, 25 an das Verbindungsstück angeformt sind. Fig. 9 zeigt, das der stufenförmige Abschnitt des Verbindungsstücks 5"" des Überleitsystems fest und dicht in dem ersten Anschlusstrichter 20 des Verbindungsstücks 7" des Sondenschlauchs sitzt.

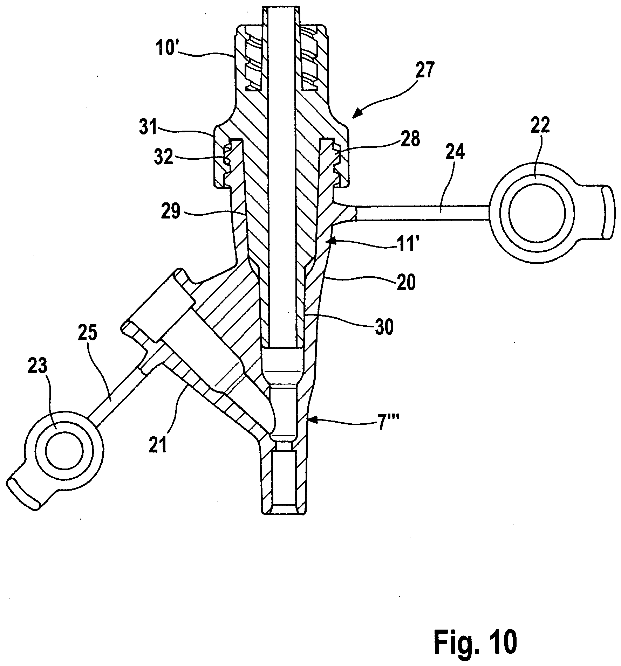

Fig. 10 zeigt ein Ausführungsbeispiel einer alternativen Ausführungsform des erfindungsgemäßen Verbindungsstücks 27 in geschnittener Darstellung zusammen mit dem Verbindungsstück 7'" des Sondenschlauchs, das wieder die beiden Anschlusstrichter 20, 21 aufweist. Das Verbindungsstück 7'" entspricht dem Verbindungsstück 7" der Fig. 9 mit der Ausnahme, das der erste Anschlusstrichter 20 von Fig. 10 ein Außengewinde 28 aufweist. Das Ausführungsbeispiel von Fig. 10 unterscheidet sich weiterhin von der Ausführungsform von Fig. 9 durch das Verbindungsstück 27, an das die Schlauchleitung des Überleitsystems angeschlossen wird.

Das Verbindungssstück 27 weist als proximales Anschlussstück für die Schlauchleitung des Überleitsystems ein Luer-Lock-Anschlussstück 10' auf, das dem Fachmann als solches bekannt ist. An das Luer-Lock-Anschlussstück 10' schließt sich das distales Anschlussstück 11' an, das mit dem ersten Anschlussstrichter 20 verbunden wird.

Das distale Anschlussstück 11' weist bei dem vorliegenden Ausführungsbeispiel nicht einen stufenförmiges, sondern einen konischen Abschnitt 29 auf, der in einen Verbindungskonus 30 übergeht, der einen kleineren Durchmesser als der konische Abschnitt hat. Der konische Abschnitt 29 des distalen Anschlussstücks sitzt in dem oberen Abschnitt des ersten Anschlusstrichters 20 mit dem größeren Innendurchmesser, während der konische Verbindungskonus 30 in dem unteren Abschnitt des Anschlusstrichters 20 mit dem kleineren Innendurchmesser sitzt. Dadurch sind beide Teile gegeneinander abgedichtet.

Zur Sicherung des Verbindungsstücks 27 an dem Anschlusstrichter 20 weist auch bei diesem Ausführungsbeispiel das distale Anschlussstück 11' ein Arretierungselement auf, das hier als eine Schraubkappe 31 mit einem Innengewinde 32 ausgebildet ist, die den konischen Abschnitt 29 des Verbindungsstücks 27 über einen Teil seiner Länge umschließt. Die Schraubkappe 31 ist mit dem Anschlusstrichter 20 verschraubt, so dass beide Teile lösbar miteinander verbunden sind.

## Patentansprüche

1. Verbindungsstück für ein enterales Überleitsystem, das ein distales Anschlussstück (11) zum Anschluss eines Verbindungsstücks eines Sondenschlauchs einer enteralen Ernährungssonde und ein proximales Anschlussstück (10) zum Anschluss des Verbindungsschlauchs eines Überleitsystems aufweist, wobei das distale Anschlussstück (11) mit mehreren Abschnitten (11A, 11B, 11C, 11D) unterschiedlichen Durchmessers stufenförmig ausgebildet ist, so dass eine lösbare Verbindung mit trichterförmigen Verbindungsstücken unterschiedlichen Durchmessers der Sondenschläuche von verschiedenen Ernährungssonden herstellbar ist,
**dadurch gekennzeichnet, dass** ein vorderster Abschnitt (11D) des distalen Anschlussstücks (11) mit dem kleinsten Durchmesser einen als Außenkonus ausgebildeten distalen Verbindungskonus (12A) aufweist, an den sich proximal ein als Arretierungselement ausgebildeter Abschnitt (12B) anschließt, wobei das Arretierungselement des distalen Anschlussstücks (11) als Schraubverbindungselement (12B) oder als Bajonett-Verbindungselement (12B') ausgebildet ist, so dass das distale Anschlussstück (11) derart ausgebildet ist,
dass einerseits eine formschlüssigen lösbare Verbindung mit einem Verbindungsstück eines Sondenschlauchs einer enteralen Ernährungssonde herstellbar ist, das ein mit dem Arretierungselement (12B) des distalen Anschlussstücks (11) komplementäres Arretierungselement aufweist, und
dass andererseits eine lösbare Verbindung mit trichterförmigen Verbindungsstücken unterschiedlichen Durchmessers der Sondenschläuche von verschiedenen Ernährungssonden herstellbar ist.

2. Verbindungsstück für ein enterales Überleitsystem nach Anspruch 1, **dadurch gekennzeichnet, dass** das Schraubverbindungselement ein zylindrisches Verbindungselement (12B) mit einem Außengewinde (13) ist.

3. Verbindungsstück für ein enterales Überleitsystem nach Anspruch 1, **dadurch gekennzeichnet, dass** das Bajonett- Verbindungselement (12B') ein zylindrisches Verbindungselement ist, dessen Mantelfläche eine Nut (13') mit einem axial verlaufenden Abschnitt (13A) aufweist, an den sich ein über einen Teil des Umfangs der Mantelfläche verlaufender Abschnitt (13B) anschließt.

4. Verbindungsstück für ein enterales Überleitsystem nach einem der Ansprüche 1 bis 3, **dadurch gekennzeichnet, dass** das proximale Anschlussstück (10) als ein Anschlussstück zur Herstellung einer lösbaren Verbindung mit einem komplementären Anschlussstück des Überleitsystems ausgebildet ist.

5. Verbindungsstück für ein enterales Überleitsystem nach Anspruch 4, **dadurch gekennzeichnet, dass** proximale Anschlussstück ein Luer-Lock- Anschlussstück (10') ist.

6. Überleitsystem für die enterale Ernährung mit einem Verbindungsstück (5) nach einem der Ansprüche 1 bis 5.

7. Überleitsystem für die enterale Ernährung nach Anspruch 6, **dadurch gekennzeichnet, dass** das Überleitsystem (1) einen Verbindungsschlauch (3) mit einem distalen und proximalen Ende aufweist, wobei das proximale Anschlusstück (10) des Verbindungsstücks (5) mit dem distalen Ende des Verbindungschlauchs fest verbunden ist.

8. Anordnung aus einem Überleitsystem nach Anspruch 6 oder 7 und einer enteralen Ernährungssonde (2), die einen Sondenschlauch (6) mit einem Verbindungsstück (7,7') zum Anschluss des Überleitsystems aufweist, **dadurch gekennzeichnet, dass** das Verbindungsstück (7, 7') des Sondenschlauchs (6) ein mit dem Arretierungselement (12B) des Verbindungsstücks (5, 5') des Überleitsystems (1) komplementäres Arretierungselement (14A, 14A') aufweist.

9. Anordnung nach Anspruch 8, **dadurch gekennzeichnet. dass** das Verbindungsstück (7. 7') des Sondenschlauchs (6) und das Verbindungsstück (5, 5') des Überleitsystems (1) als abdichtende Schraubverbindung ausgebildet sind.

10. Anordnung nach Anspruch 9, **dadurch gekennzeichnet dass** das Verbindungsstück (7''') des Sondenschlauchs (6) einen Anschlusstrichter (20) aufweist, der ein Außengewinde (28) aufweist.

## Claims

1. Connection piece for an enteral transfer system having a distal attachment piece (11) for attaching a connection piece of a probe tube of an enteral feed probe and a proximal attachment piece (10) for attaching the connection tube of a transfer system, the distal attachment piece (11) being designed stepped, having a plurality of sections (11A, 11B, 11C, 11D) with different diameters, so that a detachable connection can be effected using funnel-shaped connection pieces having different diameters from the probe tubes of different feed probes,
**characterized in that** a foremost section (11D) of the distal attachment piece (11) having the smallest diameter has a distal connection cone (12A) which is designed as an outer cone and is adjoined proximally by a section (12B) designed as a locking element, wherein the locking element of the distal attachment piece (11) is designed as a screw connection element (12B) or as a bayonet connection element (12B') or as a bayonet connection element (12B'), so that the distal attachment piece (11) is designed in such a fashion
that, firstly, an interlocking detachable connection can be effected to a connection piece of a probe tube of an enteral feed probe having a complementary locking element to the locking element (12B) of the distal attachment piece (11), and
that, secondly, a detachable connection can be effected using funnel-shaped connection pieces having different diameters from the probe tubes of different feed probes.

2. Connection piece for an enteral transfer system according to Claim 1, **characterized in that** the screw connection element is a cylindrical connection element (12B) with a male thread (13).

3. Connection piece for an enteral transfer system as claimed in Claim 1, **characterized in that** the bayonet connection element (12B') is a cylindrical connection element, the lateral surface of which has a groove (13') with a section (13A) which runs axially and which is adjoined by a section (13B) which runs over part of the circumference of the lateral surface.

4. Connection piece for an enteral transfer system as claimed in one of Claims 1 to 3, **characterized in that** the proximal attachment piece (10) is designed as an attachment piece to effect a detachable connection to a complementary attachment piece of the transfer system.

5. Connection piece for an enteral transfer system as claimed in Claim 4, **characterized in that** the proximal attachment piece is a Luer lock attachment piece (10').

6. Transfer system for enteral feeding having a connection piece (5) as claimed in one of Claims 1 to 5.

7. Transfer system for enteral feeding as claimed in Claim 6, **characterized in that** the transfer system (1) has a connection tube (3) with a distal and a proximal end, the proximal attachment piece (10) of the connection piece (5) being fixedly connected to the distal end of the connection tube.

8. Arrangement comprising a transfer system as claimed in Claim 6 or 7 and an enteral feed probe (2) having a probe tube (6) with a connection piece (7, 7') for attaching the transfer system, **characterized in that** the connection piece (7, 7') of the probe tube (6) has a locking element (14A, 14A') which complements the locking element (12B) of the connection piece (5, 5') of the transfer system (1).

9. Arrangement as claimed in Claim 8, **characterized in that** the connection piece (7, 7') of the probe tube (6) and the connection piece (5, 5') of the transfer system (1) are designed as a sealing screw connection.

10. Arrangement as claimed in Claim 9, **characterized in that** the connection piece (7''') of the probe tube (6) has a connection funnel (20) with a male thread (28).

## Revendications

1. Pièce de raccordement pour un système de transfert entéral qui présente une pièce de raccordement distale (11) pour le raccordement d'une pièce de raccordement d'un tube de sonde d'une sonde d'alimentation entérale et une pièce de raccordement proximale (10) pour le raccordement du tube de raccordement d'un système de transfert, la pièce de raccordement distale (11) étant réalisée de manière étagée avec plusieurs portions (11A, 11B, 11C, 11D) de diamètres différents de telle sorte qu'une connexion amovible avec des pièces de raccordement en forme d'entonnoir de différents diamètres des tubes de sonde de différentes sondes d'alimentation puisse être établie,
**caractérisée en ce qu'**une portion la plus en avant (11D) de la pièce de raccordement distale (11) ayant le plus petit diamètre présente un cône de raccordement distal (12A) réalisé sous forme de cône externe au niveau duquel se raccorde, en position proximale, une portion (12B) réalisée sous forme d'élément d'arrêt, l'élément d'arrêt de la pièce de raccordement distale (11) étant réalisé sous forme d'élément de raccordement à vis (12B) ou sous forme d'élément de raccordement à baïonnette (12B'), de telle sorte que la pièce de raccordement distale (11) soit réalisée de telle sorte que
d'une part un raccordement amovible par engagement par correspondance de formes puisse être établi avec une pièce de raccordement d'un tube de sonde d'une sonde d'alimentation entérale, qui présente un élément d'arrêt complémentaire de l'élément d'arrêt (12B) de la pièce de raccordement distale (11), et
d'autre part un raccordement amovible avec des pièces de raccordement en forme d'entonnoir de différents diamètres des tubes de sonde de différentes sondes d'alimentation puisse être réalisé.

2. Pièce de raccordement pour un système de transfert entéral selon la revendication 1, **caractérisée en ce que** l'élément de raccord à vis est un élément de raccordement cylindrique (12B) avec un filetage extérieur (13).

3. Pièce de raccordement pour un système de transfert entéral selon la revendication 1, **caractérisée en ce que** l'élément de raccordement à baïonnette (12B') est un élément de raccordement cylindrique dont la surface d'enveloppe présente une rainure (13') avec une portion s'étendant axialement (13A) au niveau de laquelle se raccorde une portion (13B) s'étendant sur une partie de la périphérie de la surface d'enveloppe.

4. Pièce de raccordement pour un système de transfert entéral selon l'une quelconque des revendications 1 à 3, **caractérisée en ce que** la pièce de raccordement proximale (10) est réalisée sous forme de pièce de raccordement pour établir une liaison amovible avec une pièce de raccordement complémentaire du système de transfert.

5. Pièce de raccordement pour un système de transfert entéral selon la revendication 4, **caractérisée en ce que** la pièce de raccordement proximale est une pièce de raccordement de type à verrouillage de Luer (10').

6. Système de transfert pour l'alimentation entérale avec une pièce de raccordement (5) selon l'une quelconque des revendications 1 à 5.

7. Système de transfert pour l'alimentation entérale selon la revendication 6, **caractérisé en ce que** le système de transfert (1) présente un tube de raccordement (3) avec une extrémité distale et une extrémité proximale, la pièce de raccordement proximale (10) de la pièce de raccordement (5) étant raccordée fixement à l'extrémité distale dudit tube de raccordement.

8. Agencement constitué d'un système de transfert selon la revendication 6 ou 7 et d'une sonde d'alimentation entérale (2), qui présente un tube de sonde (6) avec une pièce de raccordement (7, 7') pour le raccordement du système de transfert, **caractérisé en ce que** la pièce de raccordement (7, 7') du tube de sonde (6) présente un élément d'arrêt (14A, 14A') complémentaire de l'élément d'arrêt (12B) de la pièce de raccordement (5, 5') du système de transfert (1).

9. Agencement selon la revendication 8, **caractérisé en ce que** la pièce de raccordement (7, 7') du tube de sonde (6) et la pièce de raccordement (5, 5') du système de transfert (1) sont réalisées sous forme de raccordement à vis hermétique.

10. Agencement selon la revendication 9, **caractérisé en ce que** la pièce de raccordement (7 ''') du tube de sonde (6) présente un entonnoir de raccordement (20) qui présente un filetage extérieur (28).
